# EUROPEAN PATENT APPLICATION

(11) **EP 2 484 356 A1**
(43) Date of publication of application: **08.08.2012**
(21) Application number: 10820286.2
(22) Date of filing: 31.08.2010
(51) Int. Cl.: A61K 31/198, A21D 2/24, A23C 9/13, A23G 3/34, A23L 1/20, A23L 1/202, A23L 1/22, A23L 1/238, A23L 1/24, A23L 1/305, A23L 1/48, A23L 2/52, A61P 17/16, C12C 1/00

(54) **ORAL COMPOSITION FOR REDUCING WRINKLE FORMATION**

(30) Priority: 30.09.2009 JP 2009226076
(71) Applicant: Shiseido Company, Ltd., Chuo-ku Tokyo 104-8010 (JP)
(72) Inventor: ASHIDA, Yutaka, Yokohama-shi Kanagawa 236-0004 (JP); TAKADA, Keiko, Yokohama-shi Kanagawa 236-0004 (JP); YOKOO, Mihoshi, Yokohama-shi Kanagawa 236-0004 (JP); TOJO, Yosuke, Yokohama-shi Kanagawa 236-0004 (JP)
(74) Representative: Henkel, Breuer & Partner
(86) International application number: PCT/JP2010/064817
(87) International publication number: WO 2011/040166

(57) **Abstract**

Provided is a novel composition that acts to inhibit and/or ameliorate wrinkle formation, does not have adverse effects like those of retinoids, and is highly photostable. An oral composition for reducing wrinkle formation and an oral composition for preventing wrinkle formation are provided, said compositions containing at least one compound selected from a group comprising D-glutamic acid and derivatives and/or salts thereof. Also provided are a pharmaceutical composition and a food composition containing the aforementioned oral composition for reducing wrinkle formation or the aforementioned oral composition for preventing wrinkle formation.

## Description

### TECHNICAL FIELD

The present invention relates to an oral composition for reducing wrinkle formation and an oral composition for preventing wrinkle formation comprised of one or more compounds selected from the group consisting of D-glutamic acid and derivatives and/or salts thereof, a method for reducing wrinkle formation and a method for preventing wrinkle formation comprising a step of administering the compounds.

### BACKGROUND ART

It is known that wrinkle formation proceeds as a result of a reduced function of the dermis associated with a normal aging and photo-aging and it is aggravated due to an epidermal dysfunction such as rough skin, an oxidative stress, and an ultraviolet irradiation and the like. Conventionally, it has been believed that a promotion of a collagen production leads to an inhibition and/or an improvement of the wrinkle formation. In fact, it is known that retinoids which are derivatives of vitamin A are effective when applied onto face and forearm skins in the course of normal aging and a photoaging (Non-Patent Documents 1 and 2). However, the retinoids have adverse effects such as photosensitization and an inflammatory reaction known as a retinoid reaction (Non-Patent Document 3). In addition, the retinoids generally have a high photoreactivity and should be protected from light for the purpose of being stored stably, and they are easily decomposed, under a normal living condition, by a light transmitted into the body after being administered onto a skin.

### PRIOR ART DOCUMENTS

### Non-Patent Documents

Non-Patent Document 1: Kligman, A. M. et al., J. Am. Acad. Dermatol., 15:836 (1986)
Non-Patent Document 2: Kligman, A. M. et al., J. Am. Acad. Dermatol., 29:25 (1993)
Non-Patent Document 3: Mukherjee, S. et al., Clin. Interv. Aging, 1:327 (2006)

### DISCLOSURE OF THE INVENTION

### PROBLEM TO BE SOLVED BY THE INVENTION

Accordingly, it is required to develop a novel composition having effect on preventing and/or improving wrinkle formation which does not have an adverse effect such as those of retinoids and which is highly photostable.

### MEANS FOR SOLVING THE PROBLEM

The present invention provides an oral composition for reducing wrinkle formation comprised of one or more compounds selected from the group consisting of D-glutamic acid and derivatives and/or salts thereof.

The present invention provides an oral composition for preventing wrinkle formation comprised of one or more compounds selected from the group consisting of D-glutamic acid and derivatives and/or salts thereof.

The present invention provides a pharmaceutical composition comprising an oral composition for reducing wrinkle formation or an oral composition for preventing wrinkle formation according to the invention.

The present invention provides a food composition comprising an oral composition for reducing wrinkle formation or an oral composition for preventing wrinkle formation according to the invention.

The present invention provides a method for reducing wrinkle formation comprising a step of administering an oral composition for reducing wrinkle formation comprised of one or more compounds selected from the group consisting of D-glutamic acid and derivatives and/or salts thereof.

The present invention provides a method for preventing wrinkle formation comprising a step of administering an oral composition for preventing wrinkle formation comprised of one or more compounds selected from the group consisting of B-glutamic acid and derivatives and/or salts thereof.

In the method for reducing wrinkle formation and/or the method for preventing wrinkle formation according to the present invention, the oral composition for reducing wrinkle formation and/or the oral composition for preventing wrinkle formation may be a pharmaceutical composition.

In the method for reducing wrinkle formation and/or the method for preventing wrinkle formation according to the present invention, the oral composition for reducing wrinkle formation and/or the oral composition for preventing wrinkle formation may be a food composition.

As used herein, a "wrinkle" refers to a kind of skin trouble, and is a state in which patterns formed from linear furrows on the skin surface are concentrated on a particular region with irregularity in size and alignment.

As used herein, a "skin barrier function" is a function for maintaining the skin in a normal condition which is a function for preventing a foreign body invasion or a water loss through the skin.

As used herein, a "salt" of D-glutamic acid means any salt including a metal salt, an amine salt and the like, provided that the reducing effect on wrinkle formation of D-glutamic acid is not impaired. The metal salt may include an alkaline metal salt, an alkaline earth metal salt and the like. The amine salt may include triethylamine salt, benzylamine salt and the like.

As used herein, a "derivative" of D-glutamic acid means D-glutamic acid molecule covalently bound at its amino group, carboxyl group or side chain with any substituent group, provided that the reducing effect on wrinkle formation of D-glutamic acid is not impaired. The substituent group includes, but is not limited to, a protective group, such as N-phenylacetyl group, 4,4'-dimethoxytrityl (DMT) group, etc.; a biological macromolecule, such as a protein, a peptide, a saccharide, a lipid, and a nucleic acid; a synthetic polymer, such as a polystyrene, a polyethylene, a polyvinyl, and a polyester; and a functional group such as an ester group. The ester group may include, for example, an aliphatic ester such as methyl ester and ethyl ester, and an aromatic ester.

An amino acid has optical isomers which are L-form and D-form. A natural protein has L-amino acids bound by peptide bonds and only L-amino acids are employed excluding some exceptions such as a bacterial cell wall. Therefore, it has been considered that in a mammal including a human only L-amino acids are present and only L-amino acids are utilized (Kinouchi, T. et al., TANPAKUSHITSU KAKUSAN KOSO (PROTEIN, NUCLEIC ACID AND ENZYME), 50:453-460(2005), Lehninger Principles of Biochemistry [Vol.1] 2nd ed. , pp. 132-147 (1993), Japanese-language translation, Hirokawa Shoten Ltd., Harper's Biochemistry, Original version, 22nd ed., pp. 21-30 (1991), Japanese-language translation, Maruzen Co., Ltd.). Accordingly, only L-amino acids have mostly been employed as amino acids academically and industrially for a long time.

Exceptional cases where a D-amino acid is employed, for example, are a case of using as a raw material for an antibiotics produced by a microorganism, and, a case of a food additive employing a D-amino acid in a DL-amino acid mixture for the purpose of reducing cost of fractionating only an L-amino acid from a mixture of the L- and D-amino acids, which are obtained in equimolar amounts by synthesizing the amino acids. Nevertheless, there has been no case of using only D-amino acids industrially as bioactive substances.

D-serine and D-aspartic acid have been studied to a comparatively advanced stage because of their higher ratios of D-forms. D-Serine is localized in a cerebrum and a hippocampus, and is known to be involved in an NMDA receptor regulating factor in the brain. D-Aspartic acid is localized in a testis and a pineal body, and reported to be involved in the regulation of hormone secretion (Japanese Patent Unexamined Publication No.2005-3558). Nevertheless, the reducing and/or suppressing effect on wrinkle formation of D-glutamic acid has not been elucidated.

The reducing effect on wrinkle formation of D-glutamic acid shown in the following Examples has not been known so far. Accordingly, a composition comprising D-glutamic acid according to the present invention is a novel invention.

Recently, it was reported that ddY mice were allowed to ingest freely a 10 mM aqueous solution of a D-amino acid for two weeks and then examined for the D-amino acid concentration in each organ, which was 3 to 1000 pmol per gland in a pineal body and 2 to 500 nmol per wet gram in a brain tissue (Morikawa, A. et al., Amino Acids, 32:13-20 (2007)). Based on this, the lower limit of daily intake of D-glutamic acid contained in a composition of the present invention described below was calculated.

As indicated in Examples described below, D-glutamic acid of the present invention exhibit the reducing effect on wrinkle formation in mice when given as an oral composition at concentration of 10 mM aqueous solution. Accordingly, the amount of D-glutamic acid contained in an oral composition for reducing wrinkle formation and a food composition of the present invention may be varied in a wide range, provided that D-glutamic acid at this concentration is delivered to the fibroblasts of skin tissue in vivo. When the composition of the present invention is an internal agent, the D-glutamic acid content may be 0.0000001% by weight to 100% by weight. When the composition of the present invention is an internal agent, the D-glutamic acid content is preferably 0.000001% by weight to 80% by weight, most preferably 0.00001% by weight to 60% by weight. The lower limit of the daily dose of D-glutamic acid contained in the composition of the present invention may be 0.01 ng, preferably 0.1 ng, more preferably 1 ng per kg body weight.

The composition of the present invention may further comprise, in addition to D-glutamic acid, salts of D-glutamic acid and/or derivatives capable of releasing D-glutamic acid by drug metabolizing enzymes and the like in vivo, one or more pharmaceutically acceptable additives, provided that the reducing effect on wrinkle formation of D-glutamic acid is not impaired. Such an additive includes, but is not limited to, a diluent and an extender, a binder and an adhesive, a lubricant, a glidant, a plasticizer, a disintegrant, a carrier solvent, a buffering agent, a colorant, a flavoring agent, a sweetener, a preservative, a stabilizer, and an adsorbent, as well as other pharmaceutical additives known to those skilled in the art.

The food composition of the present invention may further comprise, in addition to D-glutamic acid, salts of D-glutamic acid and/or derivatives capable of releasing D-glutamic acid by drug metabolizing enzymes and the like in vivo, a food-industrially acceptable component, such as a seasoning, a colorant, and a preservative, provided that the reducing effect on wrinkle formation of D-glutamic acid is not impaired.

The food composition of the present invention may be any one employed conventionally as a food composition including, but not limited to, a candy, a cookie, bean paste, a French dressing, a mayonnaise, a French bread, a soy sauce, yogurt, dried seasoning powder for rice, seasoning/sauce for natto (Japanese fermented soybean), natto, and unrefined black vinegar.

### BRIEF DESCRIPTION OF THE DRAWINGS

Figure 1 is a graph showing the effect of D-glutamic acid on the skin thickening in tape-stripped mice.
Figure 2 is a graph showing the effect of D-glutamic acid on the wrinkle formation in tape-stripped mice.
Figure 3 is a graph showing the effect of D-glutamic acid on the barrier function in mice irradiated with ultraviolet rays.
Figure 4 is a graph showing the effect of D-glutamic acid on the wrinkle formation in mice irradiated with ultraviolet rays.

### DESCRIPTION OF EMBODIMENTS

Examples of the present invention described below are intended only to exemplify the invention rather than to limit the technical scope thereof. The technical scope of the invention is limited only by the description in claims.

All references cited herein are incorporated by reference in their entirety.

The following experiments were conducted after obtaining an approval by Ethical Committee of Shiseido Research Center in compliance with the guidelines of National Institutes of Health (NIH) in the United States.

### Example 1

### Experimental animal

Five-week old male HR-1 hairless mice (Hoshino Laboratory Animals, Inc.) were subjected to the experiment.

### Administration of D-Glutamic acid

Drinking water containing 10 mM D-glutamic acid was given via a water supplying bottle for four weeks. The negative control employed was drinking water containing no D-glutamic acid.

### Destruction of skin barrier

For the purpose of forming a wrinkle, a tape stripping method was conducted three times a week starting on five days after the initiation of the D-glutamic acid administration according to the method by Matsunaga et al. (Matsunaga, Y. et al., Br. J. Dermatol., 156:884 (2007)). Briefly, a 24-mm width cellophane tape (Cellotape (trade mark) , Nichiban Co. , Ltd.) cut in a length suited to the dorsal length of a hairless mouse (HR-1 strain; Hoshino Laboratory Animals, Inc., Tokyo) was adhered evenly over the entire dorsal area and then peeled off. The number of this tape adhesion/peeling off cycles was adjusted so that the degree of barrier impairment resulted in the quantity of horny layer water loss of about 4 to 8 mg/cm²/h on the basis of the measurement using a water loss meter (Meeco Inc., Warrington, PA, USA). For example when a non-treated hairless mouse was subjected to the adhesion/peeling off cycle procedures, the first treatment involved four cycles, and the second and third treatments involved each five to six cycles and the treatment in the second week and later involved six to eight cycles, whereby increasing the quantity of the horny layer water loss to about 4 to 8 mg/cm²/h. When the adhesion/peeling off cycle procedures are conducted three times a week continuously, then a wrinkle perpendicular to the median line on the dorsal area of the hairless mouse is induced on about the third week or so.

### Method for measuring skin thickening

Four weeks after the initiation of the D-glutamic acid administration, the skin thickness was measured using a thickness gauge. The measured value of the skin thickness represented as "skin thickness × 2", since the measurement was conducted by calipering the skin with the thickness gauge.

### Method for judgement of wrinkle formation

Four weeks after the initiation of the D-glutamic acid administration, the dorsal areas of the mice were photographed, and a partly modified method by Bissett et al. (Bissett, D. L. et al., Photochemistry and Photobiology, 46:367 (1987)) was employed to evaluate the degree of wrinkle formation in accordance with the judgment criteria indicated in Table 1 shown below. The wrinkle evaluation procedure was performed separately by four observers, who thereafter had a conference to make a decision for a score with regard to the degree of wrinkle formation (hereinafter just referred to as "scope")

**[Table 1]**

| Score | Criteria |
|---|---|
| 0 | No wrinkle is observed. |
| 1 | Wrinkles are shallower, shorter or fewer than score 2. |
| 2 | Shallow wrinkles are observed. |
| 3 | Wrinkles are deeper or longer than score 2. Wrinkles are shallower, shorter or fewer than score 4. |
| 4 | Shallow wrinkles are observed over the entire surface. |
| 5 | Wrinkles are deeper or longer than score 4. Wrinkles are shallower or shorter than score 6. |
| 6 | Deep and long wrinkles are observed. |
| 7 | There are increased wrinkles that are deeper and longer than score 6. Wrinkles are shallower or shorter than score 8. |
| 8 | Deep and long wrinkles are observed over the entire surface. |

### Measurement results of skin thickness

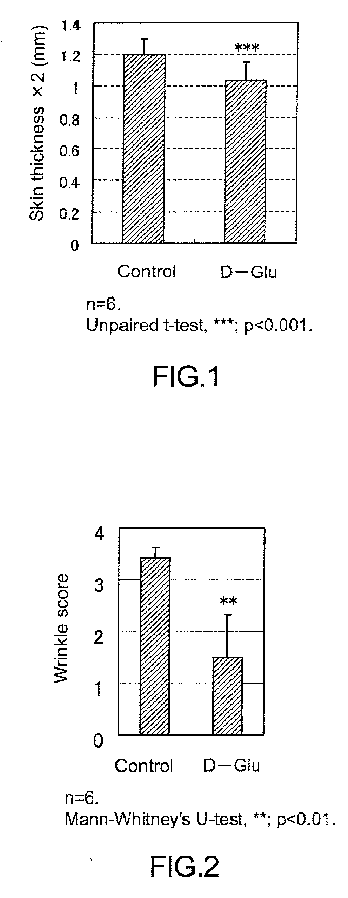

Figure 1 shows then results of the experiment examining the effect of D-glutamic acid on the skin thickening caused by a tape stripping method. The error bars for relevant experimental conditions are the standard deviations of the measured values of the results of the experiments repeated six times under identical conditions. The asterisk (***) indicates p<0.1% by Student's unpaired t-test. The mean value of the skin thickness was 1.2 in the negative control group, while it was 1.05 in the D-glutamic acid administration, group. Based on these results, it was indicated that the skin thickening caused by the tape stripping method can be statistically and significantly reduced by an administration of D-glutamic acid.

### Evaluation results of wrinkle formation

Figure 2 shows the results of the experiment examining the effect of D-glutamic acid on the wrinkle formation caused by a tape stripping method. The error bars for relevant experimental conditions are the standard deviations of the measured values of the results of the experiments repeated six times under identical conditions. The asterisk (**) indicates p<1% by Mann-Whitney's U-test. The wrinkle score in the control group was 3.5, while that in the D-glutamic acid administration group was 1.5. Based on these results, it was indicated that the wrinkle formation by the tape stripping can be statistically and significantly reduced by an administration of D-glutamic acid.

### Example 2

### Test of inhibition of wrinkle formation caused by UV-induced damage in hairless mice

### Experimental animals

Six week-old Skh-1 hairless mice (male) were employed.

### D-Glutamic acid administration

Drinking water containing 10 mM D-glutamic acid was given via a water supplying bottle for four weeks. The negative control employed was drinking water containing no D-glutamic acid.

### UV Irradiation

UV irradiation was conducted employing a partly modified method by Schwarz et al. (Haratake, A. et al., J. Invest. Dermatol. 108:769 (1997)) in accordance with the repetitive UV-B irradiation method (Naganuma, M. et al., J. Dermatol. Sci. 25:29 (2001), Schwartz, E. J., Invest. Dermatol. 91:158 (1988)). Thus, the dorsal area was irradiated with UV-B(light source: Toshiba FL-20 SE fluorescent lamp manufactured by Toshiba Electric) three times a week for four weeks. The total irradiation dose was 1.4 J/cm². The UV dose measured with UVRADIOMETER (UVR-305/365D(II), Topcon Corporation) was employed as a readout.

### Measurement of quantity of transepidermal water loss

Four weeks after the initiation of the D-glutamic acid administration, the quantity of the transepidermal water loss was measured according to a standard method.

### Measurement results of quantity of transepidermal water loss

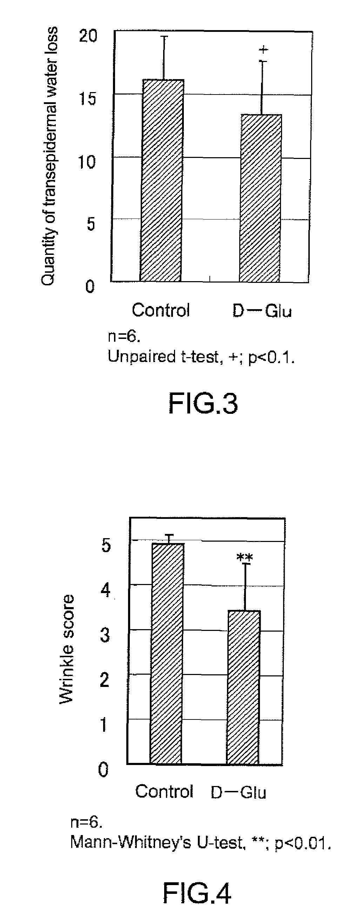

Figure 3 shows the results of the experiment examining the effect of D-glutamic acid on the skin barrier function. The error bars for relevant experimental conditions are the standard deviations of the measured values of the results of the experiments repeated six times under identical conditions. The mark (+) indicates p<10% by Student's unpaired t-test. The quantity of the transepidermal water loss in the control group was 16, while that in the D-glutamic acid administration group was 13.

### Evaluation method for wrinkle formation

The wrinkle formation was evaluated by a method similar to that in Example 1.

### Evaluation results of wrinkle formation

Figure 4 shows the results of the experiment examining the effect of D-glutamic acid on the wrinkle formation caused by the UV irradiation. The error bars for relevant experimental conditions are the standard deviations of the measured values of the results of the experiments repeated six times under identical conditions. The asterisk (**) indicates p<1% by Mann-Whitney's U-test. The wrinkle score in the control group was 4.9, while that in the D-glutamic acid administration group was 3.3. Based on these results, it was indicated that the wrinkle formation caused by the UV irradiation can be statistically and significantly reduced by an administration of D-glutamic acid.

Formulation examples of a tablet, a soft capsule, a granule, a beverage, a candy, a cookie, a bean paste, a French dressing, a mayonnaise, a French bread, a soy sauce, a yogurt, dried seasoning powder for rice, a seasoning/sauce for natto, natto, and unrefined black vinegar comprising D-glutamic acid based on the present invention are shown below. These formulation examples are for illustrative purposes only and not intended to limit the technical scope of the present invention.

### Formulation Example 1 (Tablet)

| (Composition) | Content (mg/tablet) |
|---|---|
| D-Glutamic acid | 360.5 |
| Lactose | 102.4 |
| Calcium carboxymethyl cellulose | 29.9 |
| Hydroxypropyl cellulose | 6.8 |
| Magnesium stearate | 5.2 |
| Crystalline cellulose | 10.2 |
| | 515.0 |

### Formulation Example 2 (Tablet)

| (Composition) | Content (mg/tablet) |
|---|---|
| Sucrose ester | 70 |
| Crystalline cellulose | 74 |
| Methyl cellulose | 36 |
| Glycerin | 25 |
| Sodium D-glutamate | 475 |
| N-acetylglucosamine | 200 |
| Hyaluronic acid | 150 |
| Vitamin E | 30 |
| Vitamin B6 | 20 |
| Vitamin B2 | 10 |
| α(alpha)-Lipoic acid | 20 |
| Coenzyme Q10 | 40 |
| Ceramide (Konnyaku extract) | 50 |
| L-Proline | 300 |
| | 1500 |

### Formulation Example 3 (Soft capsule)

| (Composition) | Content (mg/capsule) |
|---|---|
| Edible soybean oil | 530 |
| Eucommia ulmoides extract | 50 |
| Ginseng extract | 50 |
| Sodium D-glutamate | 100 |
| Royal jelly | 50 |
| Maca | 30 |
| GABA | 30 |
| Beeswax | 60 |
| Gelatin | 375 |
| Glycerin | 120 |
| Glycerin fatty acid ester | 105 |
| | 1500 |

### Formulation Example 4 (Soft capsule)

| (Composition) | Content (mg/capsule) |
|---|---|
| Brown rice germ oil | 659 |
| Sodium D-glutamate | 500 |
| Resveratrol | 1 |
| Lotus germ extract | 100 |
| Elastin | 180 |
| DNA | 30 |
| Folic acid | 30 |
| | 1500 |

### Formulation Example 5 (Granule)

| (Composition) | Content (mg/pack) |
|---|---|
| Sodium D-glutamate | 400 |
| Vitamin C | 100 |
| Soybean isoflavon | 250 |
| Reduced lactose | 300 |
| Soybean oligosaccharide | 36 |
| Erythritol | 36 |
| Dextrin | 30 |
| Flavoring agent | 24 |
| Citric acid | 24 |
| | 1200 |

### Formulation Example 6 (Beverage)

| (Composition) | Content (g/60 mL) |
|---|---|
| Eucommia ulmoides extract | 1.6 |
| Ginseng extract | 1.6 |
| D-glutamic acid | 0.3 |
| Reduced maltose syrup | 28 |
| Erythritol | 8 |
| Citric acid | 2 |
| Flavoring agent | 1.3 |
| N-Acetylglucosamine | 1 |
| Sodium hyaluronate | 0.5 |
| Vitamin E | 0.3 |
| Vitamin B6 | 0.2 |
| Vitamin B2 | 0.1 |
| α(alpha)-Lipoic acid | 0.2 |
| Coenzyme Q10 | 1.2 |
| Ceramide (Konnyaku extract) | 0.4 |
| L-proline | 2 |
| Purified water | Remainder |
| | 60 |

### Formulation Example 7 (Candy)

| (Composition) | Content (% by weight) |
|---|---|
| Sugar | 50 |
| Syrup | 48 |
| D-Glutamic acid | 1 |
| Flavoring agent | 1 |
| | 100 |

### Formulation Example 8 (Cookie)

| (Composition) | Content (% by weight) |
|---|---|
| Weak flour | 45.0 |
| Butter | 17.5 |
| Granulated sugar | 20.0 |
| Sodium D-glutamate | 4.0 |
| Egg | 12.5 |
| Flavoring agent | 1.0 |
| | 100.0 |

### Method for producing Formulation Example 8 (Cookie)

Granulated sugar was added in portions to butter while stirring, to which an egg, sodium D-glutamate and a flavoring agent were added and stirred. After mixing thoroughly, uniformly sieved weak flour was added and stirred at a low speed, and allowed to stand as a bulk in a refrigerator. Thereafter, it was molded and baked for 15 minutes at 170°C (degrees Celsius) to obtain a cookie.

### Formulation Example 9 (Bean paste)

| (Composition) | Content (g) |
|---|---|
| Soybean | 1000 |
| Malted rice | 1000 |
| Salt | 420 |
| D-Glutamic acid | 158 |
| Water | Remainder |
| | 4000 |

### Method for producing Formulation Example 9 (Bean paste)

Malted rice is mixed thoroughly with a salt. Washed soybeans are soaked overnight in three times its volume of water, which are then drained off, and new water is added while boiling, and poured into a colander to collect the broth (tanemizu fluid), to which D-glutamic acid is dissolved at 10% w/v. The boiled beans are minced immediately, combined with malted rice mixed with salt, to which the tanemizu fluid containing D-glutamic acid dissolved therein is added and kneaded evenly to obtain a clay-like hardness. Dumplings are made and stuffed in a container compactly without forming any voids, and the surface of the content is smoothened and sealed with a plastic film. After three months, the content is transferred to a new container and the surface is smoothened and sealed with a plastic film. Instead of adding D-glutamic acid to the tanemizu fluid, a malted rice producing a large amount of D-glutamic acid may be employed. Such malted rice can be selected by quantifying D-glutamic acid by the method described in Japanese Patent Unexamined Publication No. 2008-185558. Alternatively, a commercially available bean paste can be supplemented with D-glutamic acid or a salt thereof.

### Formulation Example 10 (French dressing)

| (Composition) | Content (g) |
|---|---|
| Salad oil | 27.7 |
| Vinegar | 30.0 |
| Sodium chloride | 0.9 |
| D-glutamic acid | 0.4 |
| Pepper | 1.0 |
| | 60.0 |

### Method for producing Formulation Example 10 (French dressing)

Vinegar is combined with sodium chloride and D-glutamic acid, and then stirred thoroughly to be dissolved. Salad oil is added to the mixture and the mixture is stirred thoroughly and then pepper is added.

### Formulation Example 11 (Mayonnaise)

| (Composition) | Content (g) |
|---|---|
| Salad oil | 134.5 |
| Vinegar | 5 |
| Sodium chloride | 0.9 |
| D-glutamic acid | 0.5 |
| Egg yolk | 18 |
| Sugar | 0.2 |
| Pepper | 0.9 |
| | 160.0 |

### Method for producing Formulation Example 11 (Mayonnaise)

An egg yolk (room temperature) is combined with vinegar, sodium chloride, D-glutamic acid and pepper, and stirred thoroughly using a whipping apparatus. Stirring is continued while adding salad oil in portions to form an emulsion. Finally, sugar is added and the mixture is stirred.

### Formulation Example 12 (French bread)

| (Composition) | Content (g) |
|---|---|
| Extra-strength flour | 140 |
| Weak flour | 60 |
| Sodium chloride | 3 |
| Sugar | 6 |
| Sodium D-glutamate | 2 |
| Dry yeast | 4 |
| Warm water | 128 |
| | 343 |

### Method for producing Formulation Example 12 (French bread)

Warm water is combined with 1g of sugar and dry yeast, which is then allowed to undergo a pre-fermentation. Extra-strength flour, weak flour, sodium chloride, 5 g of sugar and sodium D-glutamate are placed in a bowl, into which the pre-fermented yeast is placed. After kneading thoroughly into a ball-like dough, a primary fermentation is conducted at 30°C (degrees Celsius). The dough is kneaded again and allowed to stand, and then shaped into suitable forms, which are subjected to a final fermentation using an electronic fermentation machine. After forming coupes, baking is conducted for 30 minutes in an oven at 220°C (degrees Celsius).

### Formulation Example 13 (Soy sauce)

| (Composition) | Content (g) |
|---|---|
| Commercially available soy sauce | 997 |
| Sodium D-glutamate | 3 |
| | 1000 |

### Method for producing Formulation Example 13 (Soy sauce)

Commercially available soy sauce is supplemented with sodium D-glutamate, and stirred thoroughly. Instead of adding D-glutamic acid or a salt thereof, malted rice producing a large amount of D-glutamic acid may be employed for fermenting soy sauce. Such malted rice can be selected by quantifying D-glutamic acid by the method described in Japanese Patent Unexamined Publication No. 2008-185558.

### Formulation Example 14 (Yogurt)

| (Composition) | Content (g) |
|---|---|
| Milk | 897 |
| L. bulgaricus | 50 |
| S. thermophilus | 50 |
| Sodium D-glutamate | 3 |
| | 1000 |

### Method for producing Formulation Example 14 (Yogurt)

Fermentation is conducted at 40 to 45°C (degrees Celsius). Other commercially available fermentation seed organisms may be employed and commercially available yogurt may be supplemented with sodium D-glutamate. Instead of adding D-glutamic acid or a salt thereof, a seed organism producing a large amount of D-glutamic acid may be employed. Such an organism can be selected by quantifying D-glutamic acid by the method described in Japanese Patent Unexamined Publication No. 2008-185558.

### Formulation Example 15 (Dried seasoning powder for rice)

| (Composition) | Content (g) |
|---|---|
| Sodium D-glutamate | 50 |
| Laver | 15 |
| Sodium L-glutamate | 10 |
| Sodium chloride | 2 |
| Roasted sesame | 10 |
| Dried mackerel shavings | 10 |
| Sugar | 1 |
| Soy sauce | 2 |
| | 100 |

### Formulation Example 16 (Seasoning/sauce for natto)

| (Composition) | Content (g) |
|---|---|
| Commercially available sauce for natto | 9.96 |
| Sodium D-glutamate | 0.04 |
| | 10 |

### Formulation Example 17 (Natto)

| (Composition) | Content (g) |
|---|---|
| Commercially available natto | 19.9 |
| Sodium D-glutamate | 0.1 |
| | 20 |

### Method for producing Formulation Example 17 (Natto)

Instead of adding D-glutamic acid or a salt thereof, an organism producing a large amount of D-glutamic acid may be employed for producing natto. Such an organism can be selected by quantifying D-glutamic acid by the method described in Japanese Patent Unexamined Publication No. 2008-185558.

### Formulation Example 18 (Unrefined black vinegar)

| (Composition) | Content (g) |
|---|---|
| Commercially available unrefined black vinegar | 998 |
| D-glutamic acid | 2 |
| | 1000 |

### Method for producing Formulation Example 18 (Unrefined black vinegar)

Instead of adding D-glutamic acid or a salt thereof, an organism producing a large amount of D-glutamic acid may be employed for producing vinegar, black vinegar or unrefined vinegar. Such an organism can be selected by quantifying D-glutamic acid by the method described in Japanese Patent Unexamined Publication No. 2008-185558.

## Claims

1. An oral composition for reducing wrinkle formation comprised of one or more compounds selected from the group consisting of D-glutamic acid and derivatives and/or salts thereof.

2. An oral composition for preventing wrinkle formation comprised of one or more compounds selected from the group consisting of D-glutamic acid and derivatives and/or salts thereof.

3. A pharmaceutical composition comprising an oral composition according to claim 1 or 2.

4. A food composition comprising an oral composition according to claim 1 or 2.
